**Europäisches Patentamt**

**European Patent Office**

**Office européen des brevets**

(11) Veröffentlichungsnummer: **0 316 347**
**B1**

(12) **EUROPÄISCHE PATENTSCHRIFT**

(45) Veröffentlichungstag der Patentschrift: **18.07.90**

(21) Anmeldenummer: **87905205.8**

(22) Anmeldetag: **31.07.87**

(86) Internationale Anmeldenummer:
**PCT/EP87/00420**

(87) Internationale Veröffentlichungsnummer:
**WO 88/00884 11.02.88 Gazette 88/04**

(51) Int. Cl.⁵: **B 30 B 15/00**

(54) **VORRICHTUNG UND VERFAHREN ZUM KENNZEICHNEN VON PRESSLINGEN, TABLETTEN MIT LASERSTRAHLEN.**

(30) Priorität: **02.08.86 DE 3626249**
**02.08.86 DE 3626250**

(43) Veröffentlichungstag der Anmeldung:
**24.05.89 Patentblatt 89/21**

(45) Bekanntmachung des Hinweises auf die
Patenterteilung:
**18.07.90 Patentblatt 90/29**

(84) Benannte Vertragsstaaten:
**AT BE CH DE FR GB IT LI LU NL SE**

(56) Entgegenhaltungen:
**FR-A- 378 646**
**JP-A-62 164 610**
**US-A-3 657 510**

**Patent Abstracts of Japan, Band 10, Nr. 151
(M-483) (2208), 31. Mai 1986**

**Modern Plastics International, Band 11, Nr. 10,
Oktober 1981, (Lausanne, CH ), "Laser printer
codes plastics containers", siehe Seiten 21-22**

(73) Patentinhaber: **A. Nattermann & Cie. GmbH
Nattermannallee 1
D-5000 Köln 30 (DE)**

(72) Erfinder: **GAJDOS, Benedikt
Waffenschmidtstr. 2
D-5000 Köln 71 (DE)**

(74) Vertreter: **Hann, Michael, Dr. et al
Patentanwälte Dr. Michael Hann Dr. H.-G.
Sternagel Sander Aue 30
D-5060 Bergisch Gladbach 2 (DE)**

**Beschreibung**

Die Erfindung betrifft ein Verfahren und eine Vorrichtung zur Kennzeichnung von Presslingen, Tabletten mit ein- oder mehrfarbigen Symbolen, Buchstaben, Ziffern oder Bruchkerben durch Aufbringen der gewuenschten Markierungen mit einem gepulsten Laserstrahl, wobei der Laser direkt in einer Tablettenpresse angeordnet ist.

Die Kennzeichnung fester Arzneiformen ist eine Notwendigkeit, um der Verwechslungsgefahr bei der Herstellung und dem Vertrieb vorzubeugen und eine schnelle Identifikation z.B. die Vergiftungen zu ermoeglichen. So existieren z.B. in vielen Laendern der Welt diverse Empfehlungen bzw. gesetzliche Forderungen (GMP u.a.). Den Herstellern von festen Arzneimitteln obliegt die Pflicht, diese so herzustellen, dass die Verwechslungsgefahr schon beim Herstellungsprozess minimal ist. Durch Verwendung obiger Kennzeichnungen koennte die Sicherheit auf diesem Gebiet weiter erhoeht werden. Ein- oder mehrfarbige Kennzeichnung von Presslingen und Tabletten ist nach mehreren Verfahren moeglich. Ein bekanntes Verfahren der Tablettenkennzeichnung ist das Einfaerben der Pressmischung und/oder Praegung mit speziell dafuer hergestellten Praegewerkzeugen. Die Nachteile dieser Herstellung sind ein hoeherer Entwicklungsaufwand und kompliziertere Formulierungen wegen der Farbstoffverteilung und/oder Praegung. Zusaetzliche Probleme bringen gesetzliche Begrenzungen hinsichtlich der Verwendung von Farbstoffen, die international nicht immer einheitlich sind, und die durch Verwendung von Praegewerkzeugen bedingte nicht immer gewuenschte Maschinenleistung. Ausserdem sind die Praegewerkzeuge je nach Praegung deutlich teurer als Presswerkzeuge ohne Praegung. Ausserdem erfordern die mit einem Praegestempel verpressten Formulierungen eine intensivere, d.h. auch kostspielige Entwicklung, Einsatz spezieller Hilfssubstanzen, weil Tablettenformulierungen, die mit normalen, gewoelben, oder planen Stempeln problemos zu verpressen sind, beim Einsatz von Praegestempeln, je nach Beschaffenheit der Praegung, erhebliche Schwierigkeiten bereiten koennen. Im Einsatz sind auch mehrere Druckbeschriftungsverfahren und es ist z.B. im US—PS 4,548,825 eine Tintenmarkierung, sog. Ink-jet printing system, beschrieben. In allen diesen Verfahren werden die Tabletten erst nach der Herstellung in einem dazu entwickelten Geraet bedruckt, was eine wesentliche Verteuerung des Praeparates bedeutet. Ausserdem duerfte die o.g. Verwechslungsgefahr bei der Herstellung bzw. Zwischenlagerung immer vorhanden sein. Ein gaengiges Verfahren ist auch das Filmcoaten der Tabletten mit farblich unterschiedlichen Filmueberzuegen mit dem Vorteil, dass alle Praegungen, Bruchrillen usw. sichtbar bleiben. Die Tabletten fuer das Filmcoaten muessen eine gewisse minimale Woelbung aufweisen, die fuer die Rollbewegung in Coatinggeraeten wichtig ist. Der Nachteil ist, dass sich die Tabletten mit Woelbung bzw. zusaetzlicher Praegung schwieriger verpressen lassen. Im allgemeinen erfordern die Filmtabletten einen wesentlich hoeheren Rohstoff- und Entwicklungsaufwand als die Tablettenkennzeichnung durch Einfaerben der Pressmischung.

Auch das Dragieren von Tabletten ist moeglich, wobei sich aber auch hier nur bikonvexe Presslinge gut dragieren lassen und die Praegungen, Bruchrillen auf dem Pressling oder Drageekern mit der Drageehuelle ueberdeckt werden und somit nicht sichtbar bleiben. Nachteilig ist die lange Herstellungszeit und die damit verbundene Unwirtschaftlichkeit der Herstellung von Dragees.

Die Beschriftung von Tabletten mit Lasern ist grundsaetzlich bekannt (US—A—3,657,510) und Defensive Publication T 903,014). Beiden Verfahren mangelt es jedoch an einer Einrichtung zur exakten Ausrichtung der zu beschriftenden Tabletten.

Selbst bei der Annahme, dass solche Geraete schon existieren, um bei der Tablettenkennzeichnung eingesetzt zu werden, verbleibt dabei die Zwischenlagerung der zu beschriftenden Tabletten nach der Verpressung und damit das Risiko der Verwechslung gleichaussehender Tabletten vor oder bei dem Kennzeichnungsvorgang.

Aufgabe der Erfindung ist est, die Kennzeichnung von Presslingen und Tabletten sicherer zu gestalten und Verwechslungen und falsche Kennzeichnungen auszuschliessen.

Diese Aufgabe wird dadurch geloest, dass die Laserstrahlen erzeugende Einrichtung, auch einfach Laser genannt, an einer Tablettenpresse angeordnet ist und mit dieser eine Einheit bildet. Die erfindungsgemaesse Loesung ist ein Verfahren zum Aufbringen von Markierungen und/oder Bruchkerben an Presslingen oder Tabletten durch Behandeln der Oberflaeche mit einem Laserstrahl, das dadurch gekennzeichnet ist, dass die Behandlung unmittelbar vor oder nach oder waehrend des Ausstossens der Presslinges oder der Tablette aus der Matritze einer Tablettenpresse erfolgt. Vorzugsweise wird dabei der Laserstrahl auf die Oberseite des Presslings oder der Tablette umgelenkt. Bei einer anderen Ausfuehrungsform wird der Laserstrahl auf die Seitenflaeche des Presslingss oder der Tablette umgelenkt und die die Behandlung zu dem Zeitpunkt ausgefuehrt, zu dem der Pressling durch den Unterstempel aus der Matritze bis ueber deren Oberkante ausgestossen ist. Um unterschiedliche Markierungen aufbringen zu koennen, erzeugt man die Form der Markierung durch eine im Laserstrahl angeordnete Schablone mit OEffnungen in form der gewuenschten Markierung.

Die Erfindung schliesst auch eine Vorrichtung zum Anbringen von Markierungen und/oder Bruchkerben an Presslingen oder Tabletten mittels Laserstrahlen mit einer Einrichtung zum Erzeugen eines Laserstrahls ein, mit einer Einrichtung zum Erzeugen eines Laserstrahls, die an einer Tablettenpresse angeordnet und mit ihr funktionell durch Einrichtungen zu Synchronisieren verbunden ist und einer Umlenkeinrichtung, die an der Matritzenplatte der Tablettenpresse angeordnet ist, um den Laserstrahl auf die Oberflaeche des Presslings oder der der Tablette umzulenken.

Vorzugsweise ist die Einrichtung zum Erzeugen des Laserstrahls ein Gaslaser. Die Einrichtung zum Erzeugen des Laserstrahls weist eine Laseroptik mit auswechselbaren Schablonen auf, durch deren OEffnungen der Laserstrahl hindurchgeht. Die Umlenkrichtung kann seitlich neben der Matritzenplatte so angeordnet und ausgerichtet sein, dass der Laserstrahl parallel zur Oberflaeche der Matritzenplatte in Richtung auf die senkrechte Achse der Matritze verlaeuft. Alternativ wird die Umlenkeinrichtung seitlich neben dem Oberstempel der Tablettenpresse so angeordnet und ausgerichtet, dass der Laserstrahl auf die Oberseite des Presslings oder der Tablette auftrifft, ehe das Zentrum des Presslings oder der Tablette aus der senkrechten Achse des Matritze horizontal verschoben ist. Bei Excenterpressen anstelle von Rundlaufpressen ist es bevorzugt, Einrichtungen zum Hin- und Herbewegen der Umlenkeinrichtung aus einer Stellung seitlich neben dem Oberstempel in eine Stellung oberhalb der Matritze und zurueck vorzusehen, um die Markierung auf der Oberseite des Presslings anbringen zu koennen. Hervorzuheben ist dabei, dass die Zeitsynchronisation des verwendeten Lasers mit der Tablettenpresse und die durch die Tablettenpresse vorgegebene Tablettenlaufbahn ein spezielles Ausrichtungsgeraet ersetzen kann.

Die Synchronisation der Arbeitstakte der Tablettenpresse mit dem Laser ist derart, dass die Behandlung der Oberflaeche des Presslings mit dem Laserstrahl nach Abschluss des Pressvorgangs und Zurueckziehen des Oberstempels aus der Matritze erfolgt. Je nach Umlenkung des Laserstrahls trifft dieser auf die Oberseite des Presslings auf, waehrend dieser sich noch in der Matritze befindet oder waehrend des Ausstossens aus der Matritze, solange der Presslings nur vertikal, nicht jedoch horizontal bewegt wuerde.

Die Synchronisation kann aber auch so ausgebildet werden, dass ein entsprechend umgelenkter und ausgerichteter Laserstrahl auf die seitliche Oberflache des Presslings unmittelbar nach dem vertikalen Ausstoss aus der Matritze auftrifft.

Ausser den verschiedenen Zeichen, Buchstaben usw., die auf der Oberflaeche den Presslinge angebracht werden, koennen auch Markierungen seitlich auf den Raendern aufgebracht werden. Ausserdem lassen sich Teil- oder Bruchkerben auf Tabletten herstellen.

Werden Presslinge oder Tabletten mit unterschiedlichen Farbschichten mit dem Laserstrahl zu Kennzeichnung behandelt, koennen die unterschiedlichen Farbschichten je nach gewuenschter Farb- oder Bildkomposition der Markierung schichtweise ausgebrannt werden.

Die Anwendung des Systems kann damit die herkoemmliche Markierung der Presslinge durch Praegewerkzeuge ersetzen, was erherbliche Vorteile bietet, wie. z.B. Ersparnis and Praegewerkzeug, da sich mit gleichem Presswerkzeug durch Veraenderung der Zeichen bzw. Beschriftung des Lasern beliebig viele Praeparate mit einem Unterscheidungsmerkmal herstellen lassen, aber auch Ersparnis an Entwicklungsarbeiten fuer Formulierungen, da die fuer Praegestempel notwendigen aufwendigen Formulierungen entfallen.

Es kann ein handelsueblicher Gas- oder Feststoff-Laser an der Tablettpresse angeordnet werden, besonders bevorzugt sind dabei Kohlendioxid-, Exzimer- oder YAG-Laser. Dabei kann der Laserstrahl nach zwei verschiedenen Methoden (Laser Marking System) eingesetzt werden. Einmal kann der Strahl des Lasers durch eine Schablone gefuerht werden, deren OEffnungen den zu reproduzierenden Zeichen entsprechen. Zum anderen wird der Laserstrahl auf dem zu kennzeichnenden Pressling oder die Tablette fokussiert und reproduziert die gewuenschten Zeichen mit Hilfe eines computergesteuerten Programms. Der sorgfaeltig fokussierte und mit Hilfe von z.B. Glasfasern oder Spiegeln auf die Tablettenoberflaeche gefuehrte Laserstrahl bildet dann die gewuenschten Zeichen durch Schmelzen, Bleichen, Verdampfen, Oxydieren etc. der Tabletttoberflaeche. Falls notwendig, kann die Strahlenfuehrung beweglich sein und mit der Bewegung der Tablettenmaschinenteile durch z.B. teleskopische oder Schwenkbewegung synchronisiert werden. Der Laserstrahl ist intensitaetsmoduliert, so dass die fokussierte Laserenergie das zu verwendende Wort oder Symbol auf der Tablette schreibt oder druckt. Dabei ist hervorzuheben, dass diese Technik nicht auf Tabletten beschraenkt ist, sondern bei Presslingen aus unterschiedlichsten Materialien oder Zusammensetzungen Verwendung finden kann, wie. z.B. Presslinge in beliebiger Form aus verschiedenen Kunststoffen, Metallen und anderen pressfaehigen Materialien aus den Bereichen Chemie, Lebensmittel, Elektronik u.a., z.B. Bedienungsknoepfe, Katalysatoren, Keramikteile u.a.

Ein weiter Vorteil des erfindungsgemaessen Systems ist die Tatlache, dass die zu praegenden Symbole, Buchstaben oder Ziffern durch Veraenderung des Laser-Computerprogramms oder der Schriftmaske leicht ausgewechselt werden koennen.

Zuaetzliche Vorteile betreffen eine oekonomische und leistungsfaehige Produktion, da die Laserbeschriftung mit polierten und unpolierten Tabletten durchgefuehrt werden kann, weil Oberflaechenstaub auf Tabletten im Hinblick auf die Herstellung eines klaren Schriftbildes kein Problem darstellt. Das System ist daher fuer hoehere Produktionsgeschwindigkeiten in einem sauberen Verfahren geeignet, und kann darueber hinaus fuer die Kennzeichnung von unebenen, gewoelbten und anders geformten Oberflaechen verwendet werden.

Die Anordnung des erfindungsgemaessen Systems wird nun anhand der Zeichnungen noch naeher erlaeutert.

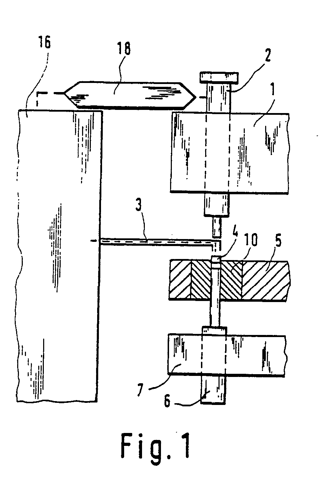

Figur 1 zeigt schematisch das erfindungsgemaesse System, bei dem ein Laser 16 an einer Tablettenpresse angeordnet und funktionell ueber ein synchronisierendes Glied 18 mit ihr verbunden ist. Die Tablettenpresse weist einen in einer Oberstempelfuehrung 2 gefuehrten beweglichen Oberstempel 1, einen in einer Unterstempelfuehrung 7 gefuehrten beweglichen Unterstempel 6 und dazwischen angeordnet den Matritzentisch 5 mit einer Matritze 10 auf. Innerhalb der Matritze ist schematisch ein

## EP 0 316 347 B1

Pressling 4 wiedergegeben, auf den der vom Laser 16 erzeugte Laserstrahl 3 gelenkt wird, um eine Markierung unmittelbar vor oder waehrend des Auswerfens des Presslings 4 aus der Matritze 10 zu bewirken.

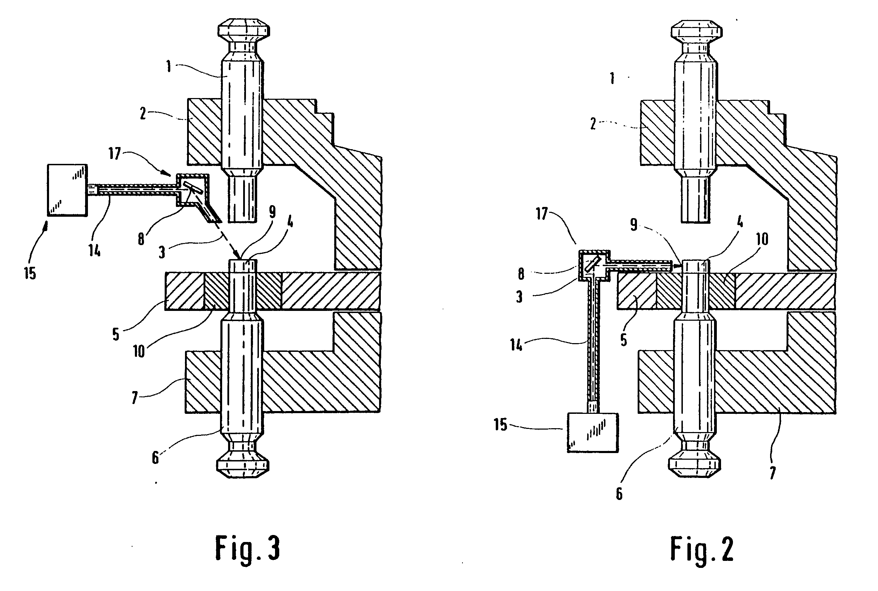

Figuren 2 und 3 zeigen die Anordnung einer mit Markierungsschablonen versehenen Laseroptik 15 im Laserstrahl 3, der mit der Einrichtung 17 zum Umlenken des Strahles mittels eines Spiegels 8 diesen auf den Presslings 4 richtet, um die Markierung 9 unmittelbar in dem Moment vorzunehmen, in dem der Pressling 4 durch den in der Stempelfuehrung 7 beweglichen angeordneten Unterstempel aus der Matrixe 10 einer Rundlaeuferpresse ueblicher Bauweise ausgestossen wird. Die Tablettenpresse weist eine Vielzahl von auf einer Kreisbahn der Matritzenscheibe 5 angeordneten Matritzen 10 auf, in die die Unterstempel 6 und der von der Oberstempelfuehrung 2 gefuehrte Oberstempel 1 eingreifen, um den Pressling 4 auszubilden. Zum Schutz verlaeuft der Laserstrahl 3 innerhalb von Schutzrohren 14. Der Laserstrahl 3 wird von einem an der Tablettenpressen angeordneten, in den Abbildungen nicht gezeigten Laser 16 erzeugt.

In Figur 2 ist die Umlenkeinrichtung 17 neben der Matritzenscheibe angeordnet, um den von unten aus dem unterhalb, wie abgebildet, oder von oben aus dem oberhalb (nicht gezeigt) der Matritzenscheibe 5 angeordneten Laser 16 auf die seitliche Kante des Presslings 4 zu lenken, so dass eine Markierung 9 auf dem Rand des Presslings 4 entsteht.

In Figur 3 sind der Laser seitlich oberhalb der Matritzenscheibe 5 und die Umlenkeinrichtung 17 mit dem Spiegel 8 direkt neben dem Oberstempel 1 angeordnet, so dass der Laserstrahl 3 auf die Oberseite des Presslings 4 im Moment des Ausstosses aus der Matritze oder gegebenenfalls auch unmittelbar vorher auftrifft und dort eine Markierung 9 erzeugt.

Um unterschiedliche Markierungen aufzubringen ist die Laseroptik 15 so ausgebildet, dass Markierungsschablonen mit OEffnungen in Form der gewuenschten Markierung oder Kennzeichnung in dem Laserstrahl 3 angeordnet werden koennen. Bei Produktwechsel ist nur ein Wechsel der Markierungschablone erforderlich.

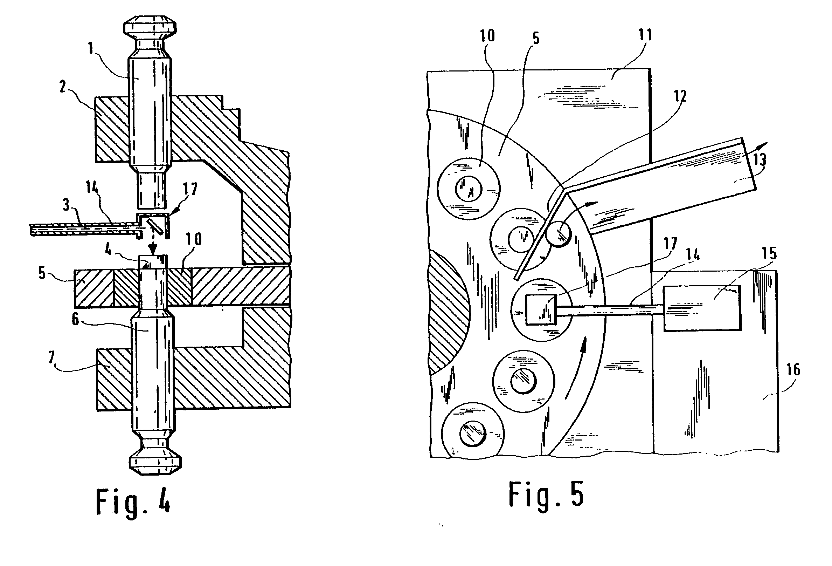

Figuren 4 und 5 zeigen die erfindungsgemaesse Ausbildung der Vorrichtung zum Markieren von Tabletten oder Presslingen mit einer Rundlaeufertablettenpresse und neben dem Maschinenkorpus 11 angeordneten Laser 16, dessen Laseroptik 15 so angeordnet ist, dass der Laserstrahl 3 im Schutzrohr 14 parallel zu Oberflaeche der Matritzenscheibe 5 im Abstand zur Oberflaeche verlaeuft. Die am Ende des Schutzrohres 14 angeordnete Umlenkeinrichtung 17 mit Spiegel 8 ist auf dem Rundlaufweg der Matritzen 10 oberhalb einer Halteposition der Matritze 10 nach dem Pressvorgang, jedoch vor dem Presslingausstoss 13 angeordnet. Dies hat den Vorteil, dass eine Kennzeichnung auf der gesamten Oberflaeche des Presslings 4 erfolgen kann und die Umlenkeinrichtung 17 und Presswerkzeuge durch Anordnung an unterschiedlichen Stellen am Rundweg der Matritze 10 innerhalb der Matritzenscheibe 5 sich raeumlich nicht behindern und das nachtraegliche Ausruesten vorhandener Pressen 16 mit Laseroptik 15 und Umlenkeinrichtung 17 erleichtern, die Presswerkzeuge sind der in der Fuehrung 2 bewegliche Oberstempel und der in der Fuehrung 7 bewegliche Unterstempel zum Eingriff in Matritzen 10.

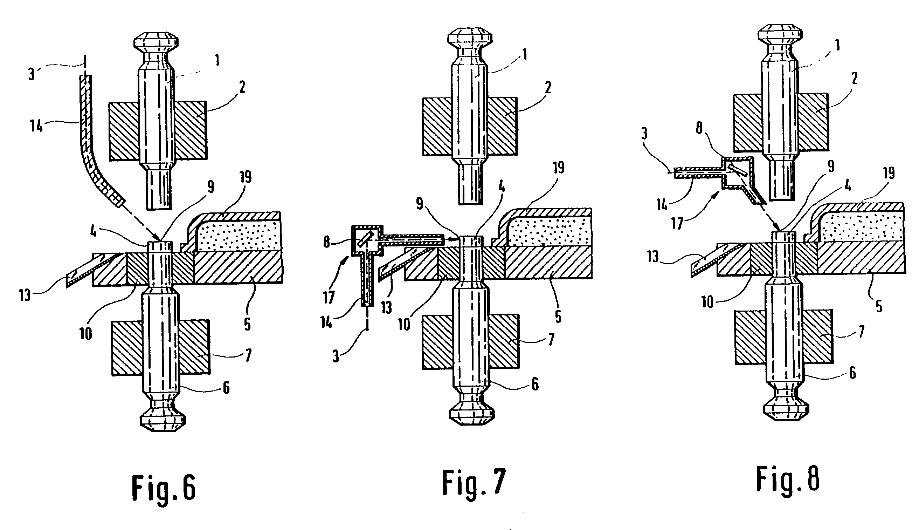

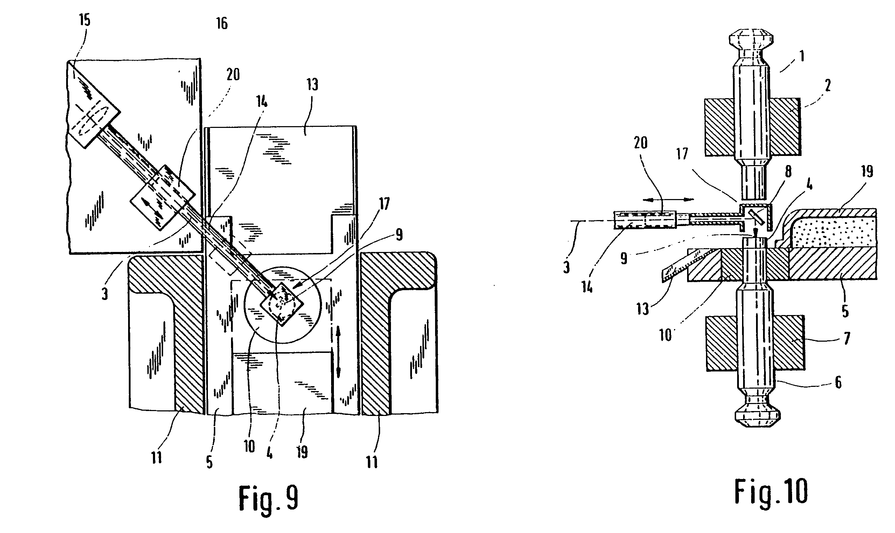

Die erfindungsgemaesse konstruktive Gestaltung ist aber auch mit sogenannten Exzenterpressen moeglich. Die Anordnung von Laser 16, Laseroptik 15 und gegebenenfalls Umlenkeinrichtung 17 ist schematisch in den Figuren 6 bis 10 wiedergegeben. Eine bekannte Konstruktion einer Exzenterpresse weist innerhalb des Maschin korpus 11 eine im Matritzentisch 5 angeordnete Matritze 10 auf, in die Oberstempel 1 und Unterstempel 6 beim Pressvorgang unter Ausbildung eines Presslings 4 eingreifen. Stempel 1 und 6 sind in Fuehrungen 2 und 7 hin-und her-beweglich angeordnet. Die Fuellung der Matritze 10 mit Material erfolgt ueber eine auf der Oberseite des Matritzentisches 5 verschiebbare Fuelleinrichtung 19, die gleichzeitig den vom Unterstempel 6 aus der Matritze 10 ausgestossenen Pressling 4 nach der Seite zur schraeg am Rande des Matritzentisches 5 angeordneten Ausstosseinrichtung 13 befoerdert. Nach der Rueckbewegung der Fuelleinrichtung ist die Presse fuer den naechsten Pressvorgang vorgebereitet. Bei Exzenterpressen ist es besonders vorteilhaft, optische Glasfasern mit entsprechender Optik als Schutzrohre 14 zur Fuehrung des Laserstrahles 3 zu verwenden, wie dies in Figur 6 gezeigt ist. Die Beweglichkeit der Glasfasern ermoeglicht eine Anordnung des Endes der Glasfaseroptik in unmittelbarer Nachbarschaft des Oberstempel 1 derart, dass der Laserstrahl auf die Oberseite des Presslings 4 zum Erzeugen einer Markierung 9 auftrifft. Alternativ kann, wie in Figur 8 gezeigt, neben dem Oberstempel auch eine Umlenkeinrichtung 17 mit Spiegel 8 angeordnet sein, um den Laserstrahl 3 auf die Oberseite des Presslings 9 zu richten, wenn dieser von Unterstempel 6 aus der Matritze 10 ausgestossen wird. Die Umlenkeinrichtung 17 mit Spiegel 8 kann aber auch, wie in Figur 7 gezeigt, seitlich am Matritzentisch 5 etwas oberhalb der Oberseite angeordnet sein, um ein Auftreffen des Laserstrahls 3 auf den seitlichen Rand des Presslings 4 und Markierung 9 an dessen Rand zu bewirken. Grundsaetzlich ist es auch moeglich, mit einer hin- und her-beweglichen Ausbildung der Umlenkeinrichtung 17 diese taktweise in eine Stellung unterhalb des Oberstempels 1 ueber den Pressling 4 zu bewegen, so dass der Laserstrahl 3 senkrecht von oben auf den Pressling 4 auftrifft, wie es in den Figuren 9 und 10 gezeigt ist. Bei entsprechender Synchronisation der Bewegungen von Oberstempel 1, Unterstempel 6 und Umlenkeinrichtung 17 kann die Markierung mittels Laserstrahl auf den Pressling 4 noch innerhalb der Matritze 10, aber auch waehrend des Ausstossvorgangs erfolgen, solange der Pressling sich noch unterhalb der in Markierungsstellung bewegten Umlenkeinrichtung 17 bzw. im Bereich des Laserstrahles 3 befindet. Die Beweglichkeit der Umlenkeinrichtung 17 kann beispielsweise durch teleskopartig ineinander vorschiebbare Schutzrohre 14 erreicht werden, die durch eine entsprechende, in den Figuren nicht gezeigte Einrichtung synchron mit den

4

Arbeitstakten der Presse bewegt werden.

Figur 9 zeigt eine solche konstruktive Gestaltung einer Exzenterpresse in Aufsicht und Figur 10 im Schnitt.

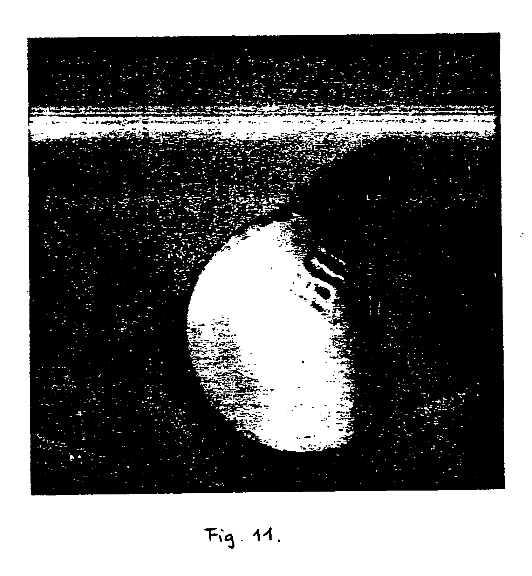

Figur 11 ist eine Fotographie einer erfindungsgemaess auf der Oberseite mit zwei Ziffern gekennzeichnet Tablette, die zeigt, dass durch den Laserstrahl in der Oberflaeche Vertiefungen erzeugt werden, die sichtbare und lesbare Markierungen darstellen.

Beispiel Herstellung von Placebokernen fuer Markierungen mit $CO_2$-Laser.

Im Prinzip kommen mehrere Stellen in der Tablettenmaschine in Frage, die fuer Tablettenmarkierung mit Laser geeignet sind. In diesem Falle wurde die Stelle ausgewaehlt, wo sich die Tablette noch in der Matritze des Drehtellers befindet; direkt vor dem Tablettenausstoss aus der Matritze. Die Synchronisation der Laserimpulse wurde durch elektronische Verbindung zwischen der Tablettenpresse und dem Laser hergestellt.

1. Zusammensetzung

| Rohstoff | pro Kern | pro Ansatz 10 kg = 30.300 Kerne a 330,0 mg |
|---|---|---|
| 1. Lactose D 80 Ph.Eur.II | 80,00 mg | 8.000,00 g |
| 2. Maisstaerke Ph.Eur.I | 10,00 mg | 1.000,00 g |
| 3. Gelatine DAB 8 | 3,00 mg | 300,00 g |
| 4. dest.Wasser | 27,00 mg | 2.700,00 g |
| 5. Maisstaerke Ph.Eur.I | 6,00 mg | 600,00 g |
| 6. Magnesiumstearat Ph.Eur.II | 1,00 mg | 100,00 g |

2. Herstellungsvorschrift

8.000,0 Lactose D 80 und 1.000,0 g Maisstaerke werden ueber ein 1,0 mm-Frewitt-Sieb gesiebt und anschliessend im Wirbelschichtgranulator Typ WSG UD 5 bei einer Zulufttemperatur von ca. 50—60°C mit der warmen Loesung aus 300,0 g Gelatine in 2.700,0 g Wasser granuliert. Nach anschliessender Siebung ueber ein 1 mm-Sieb wird das hergestellte Granulat mit anteilmaessig berechneter Menge von Maisstaerke und Magnesiumstearat abgemischt. Die so gewonnene Pressmischung wird mit Hilfe der Tablettpresse.

Typ Horn RP 16 H zu Tabletten mit folgenden Parametern verpresst:

Tablettengewicht: 330,0 mg
Tablettendurchmesser: 10,5 mm
Tablettenwoelbungsradius: 9,5 mm
Aussehen: runde, bikonvexe Filmtablettenkerne
Farbe: weiss
Zerfallzeit (in $H_2O$): ca. 10 min
Friabilitaet: max. 0,3% nach 10 min
Tablettenhaerte: ca. 80 N

3. Markierung

Die Tabletten wurden direkt nach dem Pressen mit Hilfe eines $CO^2$-Lasers in der Tablettenmaschine markiert. In die aeussere Schicht der Tablettenoberflaeche wurde die Markierung in form der Nummer "56" angebracht. Die Tablettenmaschine hat dabei die Funktion eines Ausrichtungsgeraetes angenommen.

**Patentansprueche**

1. Verfahren zum Anbringen von Markierungen und/oder Bruchkerben an Presslingen oder Tabletten durch Behandeln der Oberflaeche mit einem Laserstrahl, dadurch gekennzeichnet, dass die Behandlung unmittelbar vor oder nach oder waehrend des Ausstosses des Presslings oder der Tablette aus der Matritze einer Presse erfolgt.

2. Verfahren nach Anspruch 1, dadurch gekennzeichnet, dass der Laserstrahl auf die Oberseite des Presslings oder der Tablette umgelenkt wird.

3. Verfahren nach Anspruch 1, dadurch gekennzeichnet, dass der Laserstrahl auf die Seitenflaeche des Presslings oder der Tablette umgelenkt wird und die Behandlung zu dem Zeitpunkt erfolgt, zu dem der Pressling durch den Unterstempel aus der Matritze bis ueber deren Oberkante ausgestossen ist.

4. Verfahren nach Anspruch 1, dadurch gekennzeichnet, dass man die Form der Markierung durch eine im Laserstrahl angeordnete Schablone mit OEffnungen in Form der gewuenschten Markierung erzeugt.

5. Vorrichtung zum Anbringen von Markierung (9) und/oder Bruchkerben an Presslingen oder Tabletten (4) mittels Laserstrahlen, mit einer Einrichtung (16) zum Erzeugen eines Laserstrahls, dadurch gekennzeichnet, dass die Einrichtung (16) zum Erzeugen eines Laserstrahls (3) an einer Tablettenpresse angeordnet und mit ihr funktionell durch Einrichtungen (18) zum Synchronisieren verbunden ist und eine Umlenkeinrichtung (17) an der Matritzenplatte (5) der Tablettenpresse angeordnet ist, um den Laserstrahl (3) auf die Oberflache des Presslings der Tablette (4) umzulenken.

6. Vorrichtung nach Anspruch 5, dadurch gekennzeichnet, dass die Einrichtung (16) zum Erzeugen des Laserstrahls (3) ein Gaslaser ist.

7. Vorrichtung nach Anspruch 5, dadurch gekennzeichnet, dass die Einrichtung (16) zum Erzeugen des Laserstrahls (3) eine Laseroptik (15) mit auswechselbaren Schablonen, durch deren OEffnungen der Laserstrahl (3) tritt, aufweist.

8. Vorrichtung nach Anspruch 5, dadurch gekennzeichnet, dass die Umlenkeinrichtung (17) seitlich neben der Matritzenplatte (5) so angeordnet und ausgerichtet ist, dass der Laserstrahl parallel zur Oberflaeche der Matritzenplatte (5) in Richtung auf die senkrechte Achse der Matritze (10) verlaeuft.

9. Vorrichtung nach Anspruch 5, dadurch gekennzeichnet, dass die Umlenkeinrichtung (17) seitlich neben dem Oberstempel (1) der Tablettenpresse so angeordnet und ausgerichtet ist, dass der Laserstrahl (3) auf die Oberseite des Presslings oder der Tablette (4) auftrifft, ehe das Zentrum des Presslings oder der Tablette (4) aus der senkrechten Achse der Matritze (10) horizontal verschoben ist.

10. Vorrichtung nach Anspruch 5, dadurch gekennzeichnet, dass Einrichtungen (20) zum Hin- und Herbewegen der Umlenkeinrichtung (17) aus einer Stellung seitlich neben dem Oberstempel (1) in eine Stellung oberhalb der Matritze (10) und zurueck vorhanden sind.

## Revendications

1. Procédé pour porter des marques et/ou des entailles de rupture sur des pièces pressées ou des comprimés en traitant la surface avec un rayon laser, caractérisé en ce que le traitement se fait immédiatement avant ou après ou pendant l'éjection de la pièce pressée ou du comprimé hors de la matrice d'une presse.

2. Procédé selon la revendication 1, caractérisé en ce que le rayon laser est dévié sur la face supérieure de la pièce pressée ou du comprimé.

3. Procédé selon la revendication 1, caractérisé en ce que le rayon laser est dévié sur la surface latérale de la pièce pressée ou du coprimé et en ce que le traitement se fait à l'instant où la pièce pressée est éjectée, par l'étampe inférieure, hors de la matrice jusqu'au-delà de l'arête supérieure de cette matrice.

4. Procédé selon la revendication 1, caractérisé en ce que l'on produit la forme de la marque au moyen d'un gabarit disposée dans le rayon laser et présentant des ouvertures en forme de la marque désirée.

5. Dispositif pour porter des marques (9) et/ou des entailles de rupture sur des pièces pressées ou des comprimés (4) au moyen de rayon laser, comportant un appareil (16) poru produire un rayon laser, caractérisé en ce que l'appareil (16) de production d'un rayon laser (3) est disposé sur une presse à comprimés et lui est reliée fonctionnellement par l'intermédiaire de mécanismes (18) de synchronisation, et en ce qu'un organe de déviation (17) est disposé sur le plateau à matrices (5) de la presse à comprimés pour dévier le rayon laser (3) sur la surface de la pièce pressée ou du comprimé (4).

6. Dispositif selon la revendication 5, caractérisé en ce que l'appareil (16) de production d'un rayon laser (3) est un laser à gaz.

7. Dispositif selon la revendication 5, caractérisé en ce que l'appareil (16) de production du rayon laser (3) présente une optique de laser (15) avec des gabarits que l'on peut échanger et à travers les ouvertures desquels passe le rayon laser (3).

8. Dispositif selon la revendication 5, caractérisé en ce que l'organe de déviation (17) est disposé sur le côté, à côté du plateau à matrices (5), et orienté de façon que le faisceau laser soit dirigé parallèlement à la surface du plateau à matrices (5), en direction de l'axe vertical de la matrice (10).

9. Dispositif selon la revendication 5, caractérisé en ce que l'organe de déviation (17) est disposé sur le côté, à côté de l'étampe supérieure (1) de la presse à comprimés et orienté de façon que le rayon laser (3) tombe sur la face supérieure de la pièce pressée ou du comprimé (4) avant que le centre de la pièce pressée ou du comprimé (4) soit déplacé horizontalement en dehors de l'axe vertical de la matrice (10).

10. Dispositif selon la revendication 5, caractérisé en ce qu'il y a des appareils (20) pour donner un mouvement de va-et-vient à l'organe de déviation (17) pour passer d'une position située sur le côté, à côté de l'étampe supérieur (1), à une position située au-dessus de la matrice (10) et inversement.

## Claims

1. Process for applying markings and/or providing break notches on moulded items or tablets by treating the surface with a laser beam, characterized in that the treatment is carried out immediately before or after or during the ejection of the moulded item or the tablet from the die of a press.

2. Process as claimed in claim 1, characterized in that the laser beam is deflected onto the top of the moulded item or the tablet.

3. Process as claimed in claim 1, characterized in that the laser beam is deflected onto the side face of the moulded item or of the tablet, and the treatment takes place at the moment when the moulded item is ejected from the die by the lower punch as far as above the upper edge of the former.

4. Process as claimed in claim 1, characterized in that the form of the markings is produced by a mask arranged in the laser beam and having openings in the shape of the desired marking.

5. Device for applying markings (9) and/or providing break notches on moulded items or tablets (4) by means of laser beams and having an installation (16) which generates a laser beam, characterized in that the installation (16) for generating the laser beam (3) is arranged on a tablet-producing press, to which it is functionally connected by synchronization systems (18), and a deflection system (17) is arranged on the die support (5) of the tablet-producing press in order to deflect the laser beam (3) onto the surface of the moulded item or tablet (4).

6. Device as claimed in claim 5, characterized in that the installation (16) for generating the laser beam (3) is a gas laser.

7. Device as claimed in claim 5, characterized in that the installation (16) for generating the laser beam (3) is provided with an optical system (15) having interchangeable masks, through whose openings the laser beam (3) passes.

8. Device as claimed in claim 5, characterized in that the deflection system (17) is arranged and aligned at the side of the die support (5) in such a way that the laser beam runs parallel to the surface of the die support (5) in the direction of the vertical axis of the die (10).

9. Device as claimed in claim 5, characterized in that the deflection system (17) is arranged and aligned at the side of the upper punch (1) of the tablet-producing press in such a way that the laser beam (3) strikes the top of the moulded item or the tablet (4) before the center of the moulded item or table (4) has been displaced horizontally from the vertical axis of the die (10).

10. Device as claimed in claim 5, characterized in that systems (20) are provided to move the deflection system (17) back and forth from a position at the side of the upper punch (1) into a position above the die (10) and back again.

Fig. 1

Fig. 3

Fig. 2

EP 0 316 347 B1

Fig. 4

Fig. 5

Fig. 8

Fig. 7

Fig. 6

Fig. 9

Fig.10

Fig. 11.